# EUROPEAN PATENT APPLICATION

(11) **EP 1 700 848 A1**
(43) Date of publication of application: **13.09.2006**
(21) Application number: 06110873.4
(22) Date of filing: 08.03.2006
(51) Int. Cl.: C07D 207/38, C07C 303/40

(54) **Method for the Manufacture of Compounds Related to the Class of Substituted Sulphonyl Urea Anti-Diabetics**

(30) Priority: 11.03.2005 IN MU02712005; 27.12.2005 IN MU16272005
(71) Applicant: IPCA Laboratories Limited, 400 067 Mumbai (IN)
(72) Inventor: Kumar, Ashok, 400 053, Mumbai, Maharashtra (IN); Soudagar, Satish Rajanikant, 400 092, Mumbai, Maharashtra (IN); Mathur, Pramil Kumar, 401 107, Thane, Maharashtra (IN); Mathur, Arpana, 401 105, Thane, Maharashtra (IN); Dalavi, Dadaso Shankar, 413 102, Pune, Maharashtra (IN); Gunjal, Sanjay Tukaram, 400 067, Mumbai, Maharashtra (IN); Sawant, Uttamrao Arjunrao, 401 205, Thane, Maharashtra (IN); Saxena, Ashvini, 457 001, Ratlam, Madhya Pradesh (IN); Srivastava, Bimal Kumar, 457 001, Ratlam, Madhya Pradesh (IN); Singh, Praveen Kumar, 457 001, Ratlam, Madhya Pradesh (IN); Sankla, Jagdish, 457 001, Ratlam, Madhya Pradesh (IN); Dhurandhare, Vijay, 457 001, Ratlam, Madhya Pradesh (IN)
(74) Representative: Brand, Thomas Louis

(57) **Abstract**

A process for the formation of high purity sulphonyl urea compounds in high throughput conditions. The sulphonyl urea compounds are preferably the pharmaceutically useful anti-diabetic drugs such as Glimepiride, Gliclazide, Glipizide, Glibenclamide, Glibornuride, Glisoxepide etc, where the purity & economy of the compound are of utmost importance.

## Description

### Field of Invention:

The present invention relates to a process for the preparation of sulphonyl urea compounds of Formula (I) with high conversion rates and purity. More specifically, this invention relates to a process for the manufacture of compounds of the sulphonyl urea class of anti-diabetic pharmaceutical drugs in higher purity and yield.

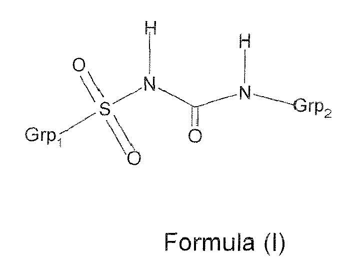

### Background of the invention:

Sulphonyl urea compounds are a class of anti-diabetic drugs which are characterized by the general structure Formula (I) which, as such or in the form of their physiologically tolerated salts, are known to have hypoglycemic properties distinguished by a powerful lowering of blood glucose level and thus find use in the treatment of diabetics.

Glimepiride is one such compound and is one of the new generation sulphonyl urea anti-diabetic molecules, and one of the best sulphonyl urea drugs in clinical appraisal. It is approved by FDA to cure diabetes type II, which cannot be cured by diet adjustment or by exercise. In general, compounds of this type lower blood glucose in non-insulin dependent diabetic mellitus (Type-II diabetic mellitus) by stimulating the release of insulin from functioning pancreatic beta cells. They may also be used along with insulin. So the synthesis of these anti-diabetic sulphonyl urea drugs in high purity is of extreme importance in the current scenario.

The pharmaceutically valuable drugs in this class include drugs such as Glisoxepid (Formula II), Glibenclamide (Formula 111), Glypinamide (Formula IV), Glimepiride (Formula V), Glibornuride (Formula VI), Glipizide (Formula Vll) and Gliclazide (Formula VIII), which are marketed for use in the therapy of type I or II diabetes mellitus. Glimepiride is currently considered to be of particular importance. The structures of these compounds are given below:

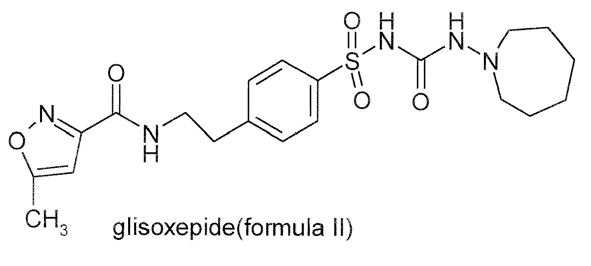

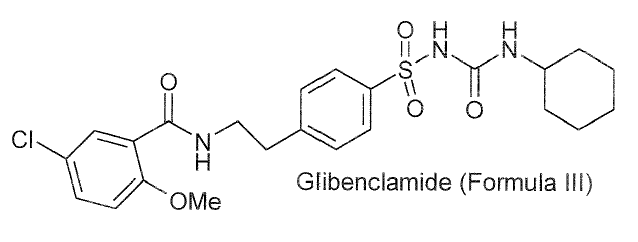

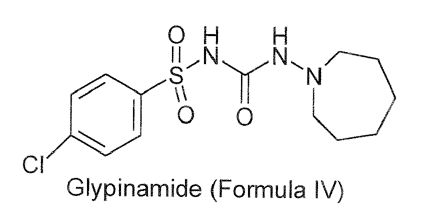

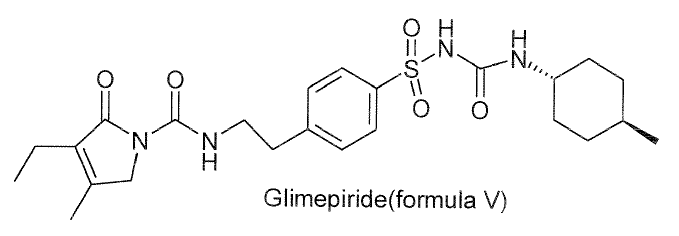

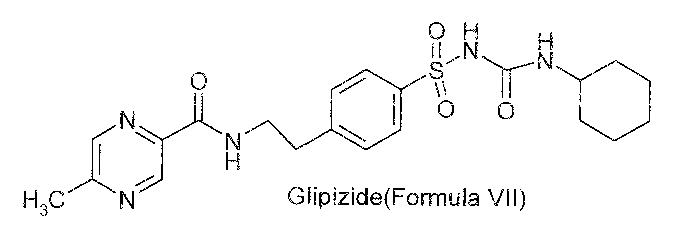

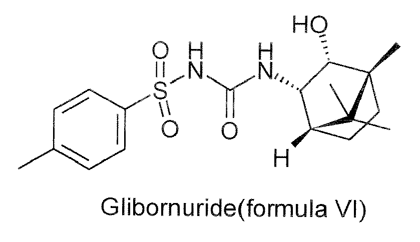

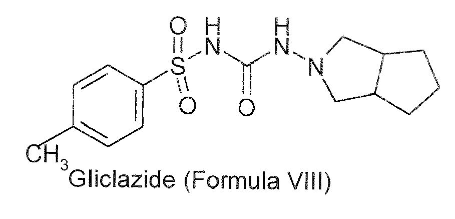

A common structural feature in many of these anti-diabetic drugs is the presence of a sulphonyl urea moiety. There are a number of known processes for providing the sulphonyl urea moiety on the compound. Many of these processes involve any one of the approaches listed below:
a) coupling of an isocyanate intermediate with sulphonamide in the presence of an inorganic base according to following scheme

   R¹SO₂NH₂ + R²NCO → R¹SO₂NHCONH-R²
b) condensation of a sulphonamide carbamate intermediate with an amino-intermediate according to following scheme

   R¹SO₂NHCOOR³+ R²NH₂ → R¹SO₂NHCONH-R²
c) Condensation of a sulphonamide compound with an activated carbamate according to the following reaction scheme

   R¹SO₂NH₂+ R²NH-COO-R⁴ → R¹SO₂NHCONH-R²

In the known processes, the resultant sulphonyl urea moiety formation was found to be incomplete resulting in poor yield and quality of these hypoglycemic pharmaceuticals due to incomplete reaction. The unreacted sulphonamide intermediates are found to contaminate the final product. Moreover the use of isocyanate poses considerable process hazards as well as handling issues when used on an industrial scale. Additionally isocyanate generates polymeric impurities during the reaction as well as under storage due to polymerization/heat instability of these reagents under the reaction conditions, resulting in the need for tedious isolation/purification procedures. Another drawback of these processes is the presence of impurities of corresponding sulphonamides either due to incomplete reaction or due to hydrolysis of the product under the reported conditions.

In the process method (c), it is observed that for transformation into sulphonyl urea, a carbamate with R⁴ having a (-)-I group seems to work, still the reaction is no longer complete. There are reports (US3787491 & US3770761) attempting the process of method (b) in the presence of bases like pyridine; it has been observed that the presence of a base does not ameliorate the above mentioned drawbacks and no added advantage is observed.

Therefore the object of this invention is to develop a rugged synthetic process for sulphonyl urea compounds, especially pharmaceutically useful anti-diabetic drugs, where purity is of utmost importance, that consists of faster reaction with complete conversions, effective purification methods and better outputs.

The present invention deals with improvements over the synthetic pathway disclosed in the prior art, which ameliorates most of the problems associated with purity and yield.

### Summary of the invention

Accordingly, there is provided a process for the formation of high purity sulphonyl urea compounds in high throughput conditions. The sulphonyl urea compounds are preferably the pharmaceutically useful anti-diabetic drugs such as Glimepiride, Gliclazide, Glipizide, Glibenclamide, Glibornuride, Glisoxepide etc, where the purity & economy of the compound are of utmost importance.

In one aspect of the present invention, there is provided a process for the preparation of sulphonyl urea compound of Formula 1, 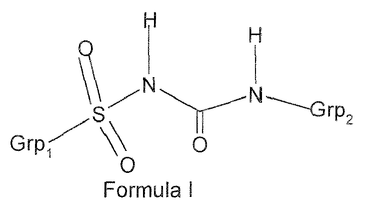 wherein Grp1 is a substituent group selected from residues A, B, C, D, E, or F given below: 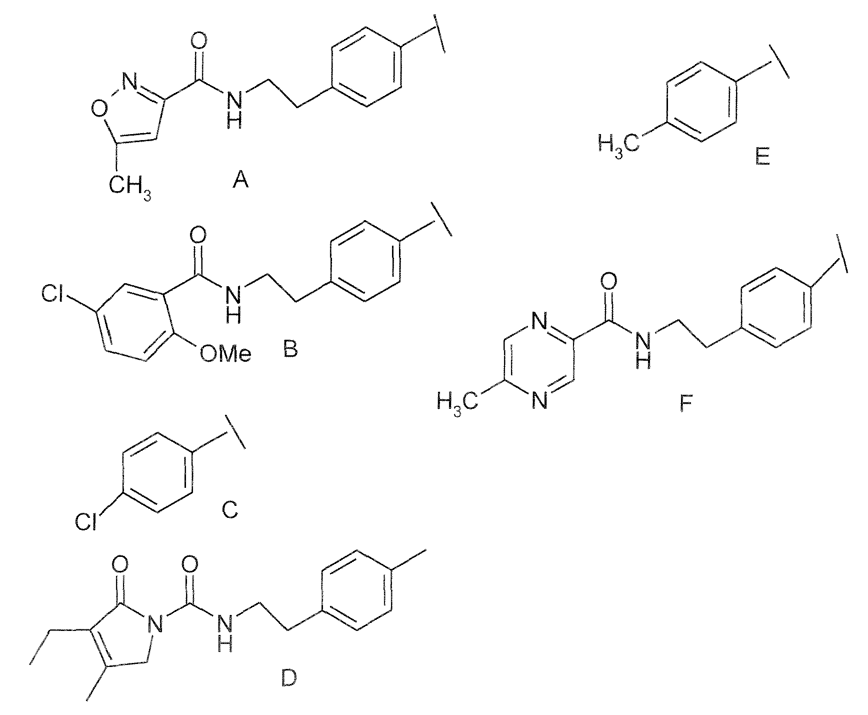 wherein Grp2 is a substituent selected from the residues P, Q, R, S, T or similar inert groups: 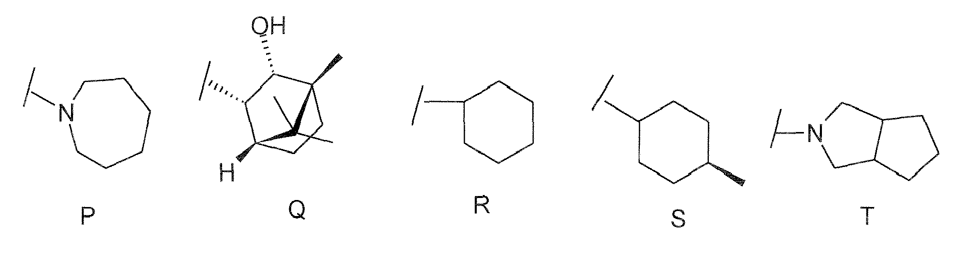 which process comprises treating a carbamate of Formula IX (wherein Grp1 is as previously defined and R' is any alkyl residue) with an amine compound of Formula (X), wherein Grp2 is as previously defined 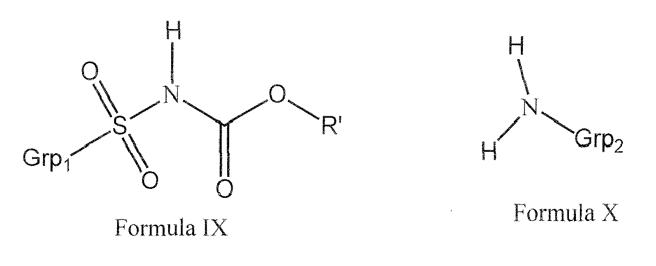 in the presence of an activation catalyst selected from 4-pyrrolidinopyridine or 4-(dimethylamino)pyridine (DMAP) or the like , in a solvent medium to form the sulphonyl urea compound (Formula I). Preferably the activation catalyst is DMAP. Preferably the solvent is a high boiling aromatic hydrocarbon solvent like toluene, and the reaction is preferably carried out at reflux temperature with optional removal of by-product (alcohol) from the reaction medium by distillation or other means like Dean Stark arrangement.

In another aspect of the present invention, there is provided a process for the manufacture of sulphonyl urea compounds of Formula I 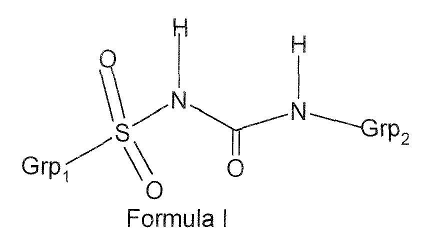 wherein, Grp1 and Grp 2 are previously defined
in higher conversion rates which process comprises treating a carbamate of Formula XII (wherein Grp2 is as defined from residue of P, Q, R, S or T and R' is any alkyl residue) with a sulphonamide of Formula XI (wherein the Grp1 is a substituent from residue A,B,C,D,E, or F) 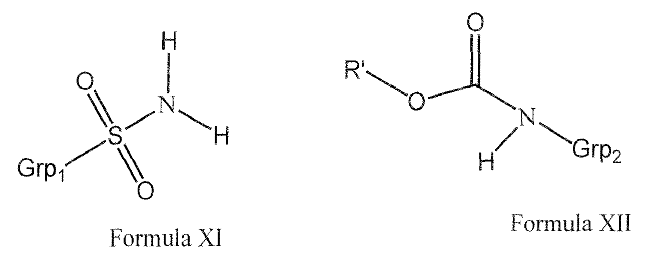 in the presence of a base and an activation catalyst selected from 4-pyrrolidinopyridine or 4-(dimethylamino)pyridine (DMAP) or the like. Suitable bases include organic or inorganic bases, and suitable combinations of two or more thereof. Furthermore the catalyst of the present invention may be used in excess to serve the purpose of the base. In such cases the catalyst is used in slight excess of equimolar quantities or more.

Various embodiments of the present invention are particularly detailed as follows.

### Detailed description;

The present process is applicable generally to the preparation of sulphonyl urea compounds of Formula (I), and in particular the preparation of high purity sulphonyl urea drugs which are useful for therapeutic applications. Some of such medicinally useful compounds are drugs such as Glimepiride, Gliclazide, Glipizide, Glibenclamide, Glibornuride, Glisoxepide etc mentioned above.

In one aspect of the present invention, an efficient process is provided for the preparation of sulphonyl urea compounds of Formula I, which is characterized by higher reaction rates and conversion rates.

The point of attachment between groups Grp1 and Grp2 is derived from a reactive synthon such as carbamate.

Therefore in one aspect of this invention, the process comprises the step of reacting a carbamate of Formula IX (as previously defined) with *an amine compound* of Formula X (as previously defined) in the presence of an activation catalyst selected from 4-pyrrolidinopyridine or 4-(dimethylamino)pyridine (DMAP) or the like. The reaction can be efficiently carried out in an organic solvent medium, preferably a non-protic organic solvent such as a hydrocarbon solvent. Preferably the activation catalyst is the DMAP. The organic solvent suitable for this conversion can be chosen from aromatic hydrocarbon solvents, saturated hydrocarbon solvents, non-polar solvents, high boiling polar aprotic solvents, and suitable combinations of two or more thereof.

Aromatic hydrocarbons can be, for example, benzene, toluene, mono-, di-, or tri-substituted benzenes, xylene and the like. High boiling polar solvents can be, for example, dioxane, dimethyl formamide, dimethyl acetamide, dimethyl sulphoxide and the like. Preferably the solvent is a high boiling aromatic hydrocarbon solvent like benzene, toluene, or xylene etc., and the reaction is preferably carried out at reflux temperature with optional removal of by-product (alcohol) from the reaction medium by distillation or any other means like Dean Stark arrangement.

The reaction is carried out by supplying heat, preferably at the boiling temperature of the solvent used. As the reaction proceeds, the by-product alcohol is accumulated in the reaction mass, which may be removed if volatile during the course of reaction.

As for the quantity of activation catalyst, preferably it is used in molar amounts ranging from 0.1 to 1.0 moles relative to the starting carbamate (IX). The most preferred amount of catalyst is in the range of about 0.3 to 0.5 moles. As for the amount of the amine compound (X), it is preferably used in molar amounts or in excess ranging from about 1.0 to 1.5 relative to the carbamate (IX) used.

The starting carbamate of Formula IX may be prepared by any suitable process such as by reacting a sulphonamide of Formula XI (as defined above) with alkyl/aryl haloformate of Formula XIII, wherein R' is an alkyl residue and halo is a halogen or pseudohalogen group: 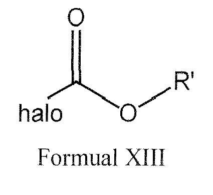 In the process, preferably the sulphonamide is reacted with alkyl/aryl haloformate in the presence of an organic base in a solvent selected from hydrocarbon and chlorinated hydrocarbon solvent. Suitable chlorinated hydrocarbons include methylene chloride, ethylene chloride, chloroform etc., and the preferred solvent is methylene chloride. Preferably the alkyl residue in the alkyl/aryl haloformate is a lower alkyl chain composed of lower alcohols, more preferably it is ethyl group and the halogen is chlorine. This makes easy removal of the by-product in the immediate inventive process at lower temperature by distillation.

The organic base used is selected from trialkyl amine such as triethylamine, ethyldiisopropyl amine or 4-(dimethylamino) pyridine (DMAP) etc., and a combination of trialkylamine, preferably triethyl amine, & DMAP is used in the reaction. The reaction is carried out at a temperature ranging from about -10 to 35°C and preferably in methylene chloride. Preferably the organic base may be used in a molar equivalent ratio relative to the sulphonamide of Formula XI. On completion of reaction, the solvent is distilled and carbamate is isolated using a second solvent like acetone or toluene. The mass is then filtered & dried which gives a purity of at least 99.5%. However, it should be noted that the isolation method may vary from one sulphonyl urea compound to other depending upon the physico-chemical properties of each compound, which method can be anything like distillation, extraction etc., a skilled artisan can make use of.

In an alternative embodiment of the present invention, a process is provided for the preparation of sulphonyl urea compounds of Formula (I) wherein the Grp1 and Grp2 are as previously defined from intermediates of XI and XII. In the intermediate compound Grp1 & Grp2, and R' group are as previously defined.

This process comprises reacting the intermediate compounds of Formula XI and XII in the presence a base material and an activation catalyst such as 4-pyrrolidinopyridine or 4-(dimethylamino) pyridine (DMAP) or the like. The reaction is efficiently carried out in the presence of the organic solvents. The organic solvents may be selected from one of those mentioned earlier for carbamate reaction. The process of this invention is characterized by higher conversion rate and faster reactions to yield the sulphonyl compounds (I) in greater yield and purity.

The base material may be selected from usual organic or inorganic bases such as metal alkoxide, metal hydroxides, trialkyl amines etc. The metal alkoxides can be, for example, sodium methoxide, sodium ethoxide, sodium/potassium tert-butoxide etc. The metal alkoxide for example can be, for example, sodium hydroxide, potassium hydroxide, sodium carbonates/potassium carbonates etc. The trialkyl amine base can be, for example, triethyl amine, disiopropylethyl amine etc. Preferably the base is a metal alkoxide like sodium methoxide.

In a preferred embodiment of the invention, the reaction is driven to completion by effectively removing the by-product, i.e., alcohol, from the reaction vessel continuously by means of distillation during the reaction. The means for effective removal of the alcohol formed in the course of reaction is preferably by distillation using a Dean-Stark arrangement or by normal distillation.

The sulphonyl urea compounds (I) obtained in the reaction are isolated in a conventional manner per se. In a typical procedure, the reaction mass after completion of reaction is cooled to a temperature of -10 to 30°C and simply filtered to obtain the crude sulphonyl urea compound (I). In most of the cases the purity of the crude product exceeds 99% (by HPLC area percent). Finally the crude sulphonyl urea compound (I) purified by precipitation from a suitable organic solvent, such as acetone and isolated pure sulphonyl urea end product in greater than 99.5 % purity (HPLC assay). However, it should be noted that, the isolation method may vary from one sulphonyl urea compound to other depending upon the physico-chemical properties of each compound, which method can be anything like distillation, extraction etc., a skilled artisan can make use of.

The starting carbamate of Formula XII is prepared from the corresponding amino compound having the Formula H2N-Grp2, wherein the Grp has substituent such as the residue P, Q, R, S, or T. The amino compound is reacted with a aryl/alkyl haloformate of Formula X in the presence of a base material in the same manner as described for carbamate of Formula IX.

The high purity pharmaceutically active sulphonyl urea compound of Formula (I) obtained by following the above-described process of the present invention, can be suitably incorporated in any conventional dosage form for administering to human patients. Apart from sulphonyl urea drug like Glimepiride, Gliclazide etc, such pharmaceutical compositions may comprise other pharmaceutically acceptable additives & excipients. Conventional dosage forms include tablets, capsules, powders, injectibles, solutions, suspensions etc.

This substantial yield and purity improvement caused by the above-described inventive variation from prior art can lead to an efficient and commercially acceptable synthetic process for the production of pharmaceutically valuable sulphonyl urea compounds.

### Examples:

### Example 1: Preparation of carbamate of Formula IX (R'= ethyl, and Grp1=D)

In a reaction vessel 100 gm of 4[2-(3-ethyl-4-methyl-2-carbonyl pyrrolidine amido) ethyl] benzene sulfonamide, 72 gm triethyl amine and 1.6 lit dichloromethane were taken and the mixture cooled to 0°C. 46.15 gm of ethyl chloroformate was diluted separately with 200 ml dichloromethane and added into the reaction vessel drop wise while maintaining the temperature at around 5°C and maintained for 2 hours at 5°C and then at 25 to 30°C till completion of reaction. 2 L water and 0.7 L dichloromethane was added and the pH of the reaction mass were adjusted to 4 by addition of acetic acid. The organic layer was separated and washed with water and concentrated to dryness. The residue was refluxed with 300 ml acetone and cooled to 25 to 30°C, maintained for 1 hour, filtered and washed with 100 ml chilled acetone to get 100 gm carbamate of Formula IX (yield 83%, purity 99.7%). Melting Point: 177 to 182°C

### Example 2: Preparation of Glimepiride (Formula I, Grp1=D and Grp2= R)

In a reaction vessel, 80 gm of carbamate of Formula IX, 27.2 gm *trans 4-*methylcyclohexyl amine, 11.5 gm 4-dimethylamino pyridine and 1.6 lit. toluene was taken and the mixture was heated to reflux and toluene was distilled out maintaining total volume of the reaction constant. After completion of reaction the mass was cooled to 25 to 30°C, to precipitate the Glimepiride, filtered and washed with 800 ml of toluene. The filtered material was dried to get 88 gm (95% yield) of Glimepiride (purity 99.5%) 80 gm of Glimepiride, obtained as per the above example, was stirred with 800 ml of acetone at reflux temperature for 30 minutes and cooled to 25 to 30°C, filtered, washed with 400 mi acetone. The filtered material was dried to weigh 75 gm Glimepiride of 99.7% purity (by HPLC) and impurity of sulphonamide (Vl) and carbamate (IX) 0.2 and 0.05 % respectively. Melting point: 207 to 211°C.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A process for making a compound of Formula (I): 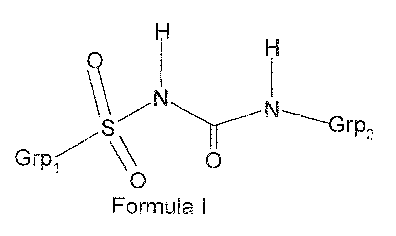 wherein Grp1 is any appropriately substituted or unsubstituted aryl residue and Grp2 is any (un)substituted cycloalkyl, polycyclic ring or heterocyclic ring, the process comprising:
EITHER:
reacting a combination of compound of formula IX and an amino-compound of Formula X, wherein Grp₁ and Grp₂ are as defined above and R' is an alkyl residue: 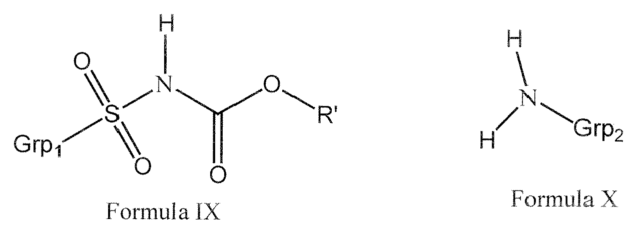
OR:
reacting a combination of a sulphonamide of Formula Xt and a carbamate of Formula XII, wherein Grp₁, Grp₂ and R' are as defined above: 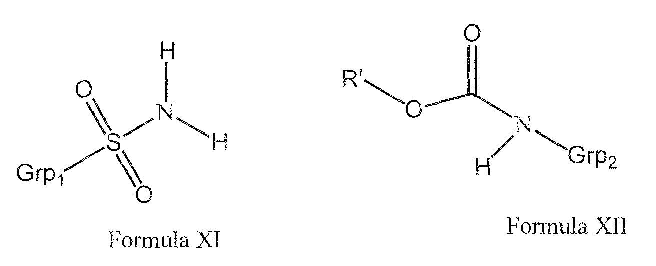
in the presence of 4-pyrrolidinopyridine or 4-(dimethylamino)-pyridine (DMAP).

2. A process according to claim 1, wherein said reaction is performed in a solvent media

3. A process according to claim 2, wherein said reaction medium comprises an organic solvent.

4. A process according to claim 3, wherein the solvent is selected from hydrocarbon solvents, non-polar solvents, or polar aprotic solvents.

5. A process according to claim 4, wherein the solvent is selected from benzene, toluene, mono, di, or tri-substituted benzenes, xylene, dioxane, dimethyl formamide, dimethyl acetamide, dimethyl sulphoxide or the like.

6. A process according to any one of claims 1 to 5, wherein said Grp1 is any one of the substituent group selected from residue A, B, C, D, E or F given below: 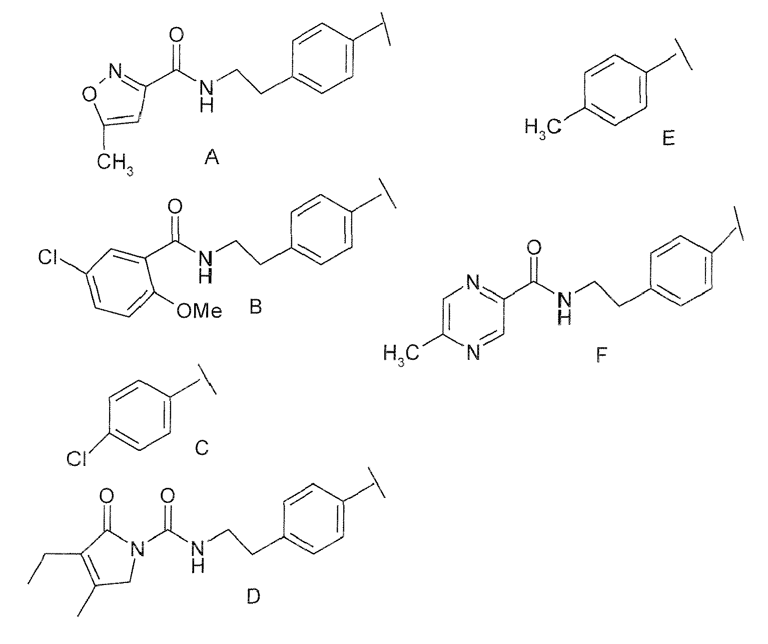 and said Grp2 is any one of the substituent group selected from residues P, Q, R, S, or T given below:

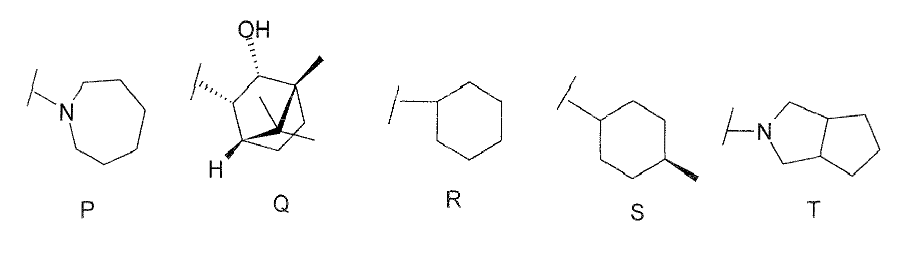

7. A process according to any one of claims 1 to 6, wherein said reaction of sulphonamide of Formula XI with carbamate of formula XII is in the presence of an additional base which is either an organic or inorganic base.

8. A process according to claim 7, wherein said base comprises an alkali metal hydroxide, an alkali metal carbonate or an alkali metal alkoxide.

9. A process according to claim 8, wherein said base is sodium methoxide or potassium carbonate.

10. A process according to any one of claims 1 to 9, wherein a by-product alcohol is continuously removed from the reaction medium.

11. A process according to any one of claims 1 to 10, for making Glisoxepide, Glibenclamide, Glypinamide, Glimepiride, Glibornuride, Glipizide or Gliclazide.

12. A process according to claim 11, for making Glimepiride.

13. A process according to any one of claims 1 to 12, wherein said starting carbamate of Formula lX is obtained by a process comprising:
combining a corresponding sulphonamide of Formula XI, wherein Grp1 is as previously defined, with a haloformate compound of Formula XIII, wherein R' is any alkyl residue 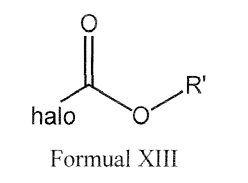 and reacting said combination in the presence of an organic base in a solvent medium selected from a hydrocarbon solvent or a chlorinated hydrocarbon solvent.

14. A process according to claim 13, wherein the organic base comprises a trialkyl amine.

15. A process according to claim 13, wherein the base is selected from triethylamine, ethyldiisopropyl amine or 4-(dimethylamino)pyridine (DMAP), or a mixture of two or more thereof.

16. A process according to claim 15, wherein said organic base is a combination of triethylamine and 4-(dimethylamino)pyridine (DMAP).
